# EUROPEAN PATENT APPLICATION

(11) **EP 3 769 735 A1**
(43) Date of publication of application: **27.01.2021**
(21) Application number: 19770915.7
(22) Date of filing: 15.03.2019
(51) Int. Cl.: A61F 13/533, A61F 13/532, A61F 13/535, A61F 13/536

(54) **ABSORBENT ARTICLE**

(30) Priority: 23.03.2018 JP 2018055517
(71) Applicant: DAIO PAPER CORPORATION, Shikokuchuo-shi Ehime 799-0492 (JP)
(72) Inventor: ODA, Terumitsu, Sakura-shi, Tochigi 329-1411 (JP)
(74) Representative: Epping - Hermann - Fischer
(86) International application number: PCT/JP2019/010996
(87) International publication number: WO 2019/181813

(57) **Abstract**

A core press and a lateral absorber embossment are formed at least on a surface of an absorber facing an upper-side sheet. The core press is formed by depressing the absorber toward a back-side sheet such that spaces are formed between the absorber and the upper-side sheet and the thickness of the absorber is reduced in the thickness direction. The lateral absorber embossment is formed in a central portion in the width direction of the absorber and extends substantially in the width direction. A non-pressed part is formed along the periphery of the lateral absorber embossment by omitting the core press in a position where the core press overlaps the lateral absorber embossment and in the vicinity of the lateral absorber embossment. A longitudinal surface embossment is formed on an outer surface of the upper-side sheet by depressing the upper-side sheet and the absorber together toward the back-side sheet such that the longitudinal surface embossment extends in the longitudinal direction in a central portion in the width direction of the absorbent article and crosses the lateral absorber embossment.

## Description

### TECHNICAL FIELD

The present invention relates to an absorbent article mainly used for products such as sanitary napkins, pantiliners, incontinence pads, and toiletries.

### BACKGROUND ART

There is a known absorbent article where an absorber formed of, for example, cotton pulp is disposed between an impermeable back-side sheet such as a polyethylene sheet or a polyethylene-sheet-laminated nonwoven fabric and a permeable upper-side sheet such as a nonwoven fabric or a permeable plastic sheet.

A slim absorbent article including a thin absorber has such advantages that wearing discomfort can be reduced and that the bumps of the absorbent article are less noticeable from the outside of clothes when the absorbent article is worn (i.e., the absorbent article has less influence on the outer appearance), and is therefore popular in the market.

However, in such a slim absorbent article, it is pointed out that the absorption rate of a body fluid is lowered when the thickness of the absorber is reduced by press processing in which the absorber is compressed in the thickness direction. Generally, the mechanism that an absorber contacts and absorbs a body fluid is that the body fluid first adheres to the surfaces of fibers of, for example, pulp constituting the absorber, and then penetrates into the absorber through gaps between the fibers (inter-fiber gaps) due to the capillary action. Therefore, the water absorption of an absorber is closely related to the water absorption of the pulp itself, the wettability of fiber surfaces, as well as the structure of the absorber such as the composition and density of fibers. When the same type of pulp is used, it is assumed that the water absorption increases as the fiber density decreases and the inter-fiber gap becomes larger. Therefore, the initial absorption rate of the body fluid is slower in a portion where the fibers are densely compressed by the press processing due to the small inter-fiber gap.

A slim absorbent article has a small absorption capacity per unit area and a low liquid retention capacity in the thickness direction because of the small thickness of the absorber. Accordingly, it is desired to efficiently use the inherent absorbent capacity of the absorber by diffusing the body fluid in the plane direction.

As a technology for improving such diffusivity, Patent Document 1 discloses an absorbent article where a non-fiber part formed of a hole passing through an absorbent core in the thickness direction is formed such that the non-fiber part extends in the longitudinal direction through the crotch. Also, Patent Document 2 discloses an absorbent article where multiple high-density parts and multiple low-density parts are formed to extend along the longitudinal direction of an absorber, and the high-density parts and the low-density parts are formed alternately in the width direction of the absorber.

### [Related-Art Documents]

### [Patent Documents]

[Patent Document 1] Japanese Laid-Open Patent Publication No. 2016-112081
[Patent Document 2] Japanese Laid-Open Patent Publication No. 2010-136899

### DISCLOSURE OF INVENTION

### PROBLEMS TO BE SOLVED BY THE INVENTION

However, with the absorbent article described in Patent Document 1, the body fluid tends to flow rapidly to the rear side through the non-fiber part. Therefore, the body fluid tends to reach the rear end of the non-fiber part, accumulate in a part of the absorber, and cause leakage. Also, when a non-fiber part is formed in the absorber of a slim absorbent article, the capacity of the absorber to absorb the body fluid decreases, and the absorption performance may be reduced.

Also, with the absorbent article described in Patent Document 2, because the high-density parts and the low-density parts are formed alternately, the thickness of the absorber tends to increase. Accordingly, this configuration is not suitable for a slim absorbent article. Further, because the high-density parts extend in the longitudinal direction of the absorber, the body fluid tends to diffuse up to the ends of the high-density parts through the high-density parts and may leak from the ends of the absorber.

For the above reasons, the main object of the present invention is to provide an absorbent article configured to efficiently use a wide area of an absorber without reducing the absorption rate and causing leakage.

### MEANS FOR SOLVING THE PROBLEMS

To solve the above problems, an aspect of the present invention provides an absorbent article that includes an absorber disposed between a permeable upper-side sheet and an impermeable back-side sheet. A core press and a lateral absorber embossment are formed at least on a surface of the absorber facing the upper-side sheet, the core press is formed by depressing the absorber toward the back-side sheet such that spaces are formed between the absorber and the upper-side sheet and the thickness of the absorber is reduced in the thickness direction, the lateral absorber embossment is formed in a central portion in the width direction of the absorber and extends substantially in the width direction, a non-pressed part is formed along the periphery of the lateral absorber embossment by omitting the core press in a position where the core press overlaps the lateral absorber embossment and in the vicinity of the lateral absorber embossment, and a longitudinal surface embossment is formed on an outer surface of the upper-side sheet by depressing the upper-side sheet and the absorber together toward the back-side sheet such that the longitudinal surface embossment extends in the longitudinal direction in a central portion in the width direction of the absorbent article and crosses the lateral absorber embossment.

### ADVANTAGEOUS EFFECT OF THE INVENTION

As described above, an embodiment of the present invention makes it possible to efficiently use a wide area of an absorber without reducing the absorption rate and causing leakage.

### BRIEF DESCRIPTION OF THE DRAWINGS

FIG. 1 is a partially cutaway view of a sanitary napkin 1 according to the present invention;
FIG. 2 is a cross-sectional view taken along line II-II of FIG. 1;
FIG. 3 is a cross-sectional view taken along line III-III of FIG. 1;
FIG. 4 is a plan view of an absorber 4;
FIG. 5 a cross-sectional view taken along line V-V of FIG. 4;
FIG. 6 is a plan view of an absorber 4 according to a variation; and
FIG. 7 is a cross-sectional view taken along line VII-VII of FIG. 4 (a vertical cross-sectional view of the absorber 4) illustrating the flow of a body fluid.

### DESCRIPTION OF EMBODIMENTS

Embodiments of the present invention are described below with reference to the accompanying drawings.

### <BASIC STRUCTURE OF SANITARY NAPKIN>

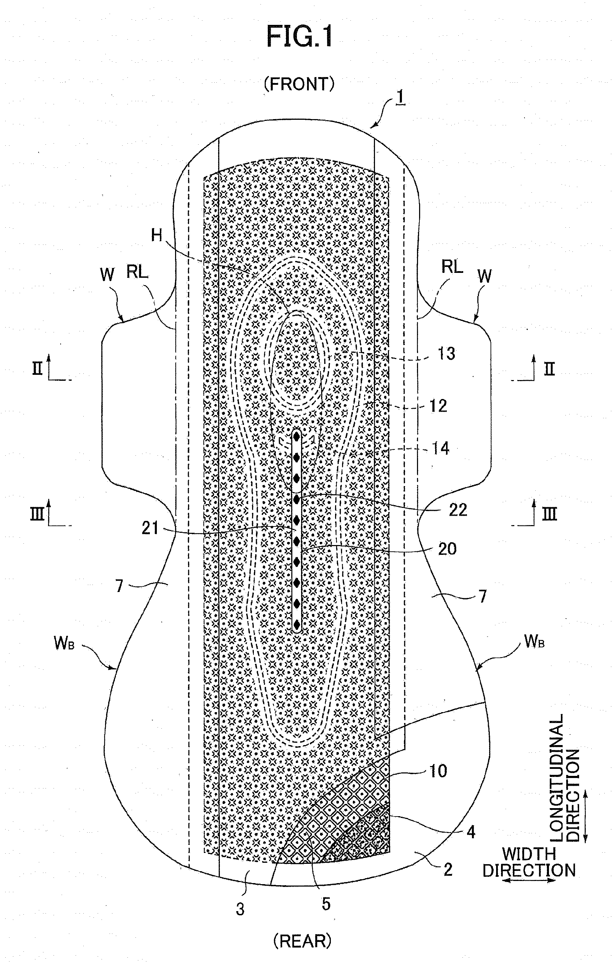

As illustrated in FIGs. 1 through 3, a sanitary napkin 1 according to the present invention includes an impermeable back-side sheet 2 formed of, for example, a polyethylene sheet, a permeable upper-side sheet 3 that allows, for example, a menstrual blood and a vaginal discharge (which are hereinafter collectively referred to as a "body fluid") to smoothly pass through, an absorber 4 formed of, for example, cotton pulp or synthetic pulp and disposed between the sheets 2 and 3, and side nonwoven fabrics 7 that are provided on the lateral sides of a skin-contact surface and extend along substantially the entire length in the longitudinal direction. In the periphery of the absorber 4, at the edges in the longitudinal direction of the absorber 4, the outer edges of the back-side sheet 2 and the upper-side sheet 3 are bonded together with an adhesive such as a hot melt or a bonding method such as heat sealing or ultrasonic sealing. Further, at side edges of the absorber 4, portions of the back-side sheet 2 and the side nonwoven fabrics 7 extending laterally beyond the absorber 4 are bonded to each other with an adhesive such as a hot melt or by a bonding method such as heat sealing or ultrasonic sealing to form flaps where the absorber is not present. In the illustrated example, to keep the shape of the absorber 4 and improve the diffusivity, the absorber 4 is enveloped in an enveloping sheet 5 formed of, for example, crepe paper or a nonwoven fabric. However, the enveloping sheet 5 may be omitted. Also, although not shown, a second sheet formed of, for example, a hydrophilic nonwoven fabric and having substantially the same shape as the upper-side sheet 3 may be provided adjacent to the non-skin side of the upper-side sheet 3.

Below, the structure of the sanitary napkin 1 is described in more detail. The back-side sheet 2 may be formed of a sheet material such as polyethylene having at least water impermeability. The back-side sheet 2 is preferably formed of a material having moisture permeability to prevent stuffiness. As the water-impermeable and moisture-permeable sheet, a microporous sheet is preferably used. A microporous sheet is obtained by melt-mixing an inorganic filler with an olefin resin such as polyethylene or polypropylene to form a sheet, and by stretching the sheet uniaxially or biaxially. On the non-skin side (outer surface) of the back-side sheet 2, one or more streaks of adhesive layers (not shown) are formed along the napkin longitudinal direction to fix the sanitary napkin 1 to underwear when the sanitary napkin 1 is worn on the body. The back-side sheet 2 may also be implemented by a polyethylene-laminated nonwoven fabric (a nonwoven fabric laminated with a polyethylene film) that is formed by stacking a plastic film and a nonwoven fabric.

The upper-side sheet 3 is preferably made of a porous or nonporous nonwoven fabric or a porous plastic sheet. Examples of fiber materials of the nonwoven fabric include synthetic fibers formed of olefin such as polyethylene or polypropylene, polyester, or polyamide, regenerated fibers such as rayon and cupra, and natural fibers such as cotton. The nonwoven fabric may be produced by any appropriate production method such as spunlacing, spunbonding, thermal bonding, melt-blowing, or needle punching. Among these production methods, spunlacing has an advantage in terms of flexibility and draping characteristics, and thermal bonding has an advantage in terms of bulkiness and compression restoration. When a large number of through holes are formed in the upper-side sheet 3, the sanitary napkin 1 can quickly absorb a body fluid and its dry touch property is improved. Although either long fibers or short fibers may be used for the nonwoven fabric, short fibers are preferably used to give a texture of a towel cloth. Also, to facilitate embossing, olefin fibers made of, for example, polyethylene or polypropylene with a relatively low melting point are preferably used. Also, bicomponent fibers may be used for the nonwoven fabric. Examples of bicomponent fibers include a core-in-sheath fiber including a high-melting-point fiber as a core and a low-melting-point fiber as a sheath, a side-by-side fiber, and a split fiber.

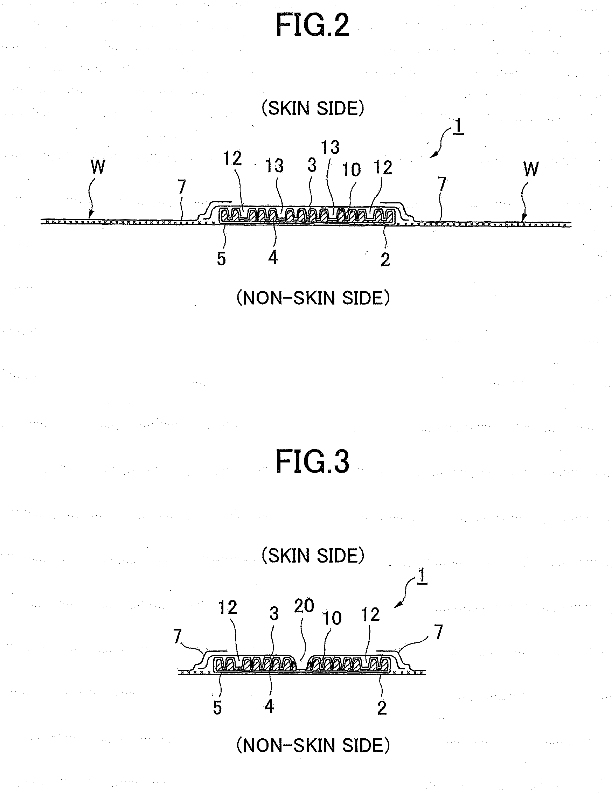

The width of the upper-side sheet 3 is made slightly greater than the width of the absorber 4 as illustrated in the cross-sectional views of FIGs. 2 and 3 such that the upper-side sheet 3 just covers the absorber 4. The side nonwoven fabrics 7 different from the upper-side sheet 3 are provided outside of the absorber 4. The side nonwoven fabrics 7 are formed of a non-woven fabric material on which a water repellent treatment or a hydrophilic treatment is performed depending on a purpose such as preventing penetration of menstrual blood or a vaginal discharge or improving the feel.

The side nonwoven fabrics 7 may be produced using natural fibers, synthetic fibers, or regenerated fibers as a material according to any appropriate production method. A nonwoven fabric having a reduced basis weight and breathability is preferably used to reduce coarseness and prevent stuffiness. Specifically, a nonwoven fabric with a basis weight of 13-23 g/m² is preferably used. Also, a water-repellent non-woven fabric coated with, for example, a silicone-based, paraffin-based, or alkylchromic chloride-based water repellent is preferably used to reliably prevent the permeation of a body fluid.

As illustrated in FIG. 2, an outer portion of each of the side nonwoven fabrics 7 located outside of a middle portion in the width direction is bonded with an adhesive such as a hot melt across a section from a predetermined inner position to an outer edge of the back-side sheet 2 to form a predetermined flap at a predetermined position. The flaps may form right and left wing flaps W on the sides of a portion H that substantially corresponds to a body fluid discharge part as well as hip hold flaps W_{B} on the buttock side (rear side). An adhesive layer (not shown) may be provided on the outer side of each of the wing flaps W and the hip hold flaps W_{B}. When the sanitary napkin 1 is attached to the shorts, the wing flaps W are folded back to the opposite side along folding lines RL at the base ends and wrapped around and fixed to the crotch of the shorts, and the hip hold flaps W_{B} are fixed to the inner surface of the shorts. On the other hand, the inner portions of the side nonwoven fabrics 7 are stacked on the skin side of the upper-side sheet 3 and bonded to the absorber 4 (the skin side of the upper-side sheet 3).

### <ABSORBER>

The absorber 4 disposed between the back-side sheet 2 and the upper-side sheet 3 is formed of, for example, pulp and a superabsorbent polymer. The superabsorbent polymer is mixed in the pulp constituting the absorber in the form of, for example, granular powder. The pulp may be made of cellulose fibers such as chemical pulp or dissolving pulp made from wood, or synthetic cellulose fibers such as rayon or acetate. In terms of the function and the price, softwood pulp with a long fiber length is more preferable than hardwood pulp.

The sanitary napkin 1 is a thin slim napkin using the absorber 4 with a low basis weight. The basis weight of the pulp is between 50 g/m² and 300 g/m² and preferably between 80 g/m² and 220 g/m², and the basis weight of the superabsorbent polymer is between 30 g/m² and 180 g/m² and preferably between 50 g/m² and 160 g/m². The basis weights of the pulp and the superabsorbent polymer are not necessarily constant, and may be changed depending on the portions of the absorber. For example, a high absorption part where the basis weights of the pulp and the superabsorbent polymer are high may be formed in an area H corresponding to the body fluid discharge part of a wearer. To reduce wearing discomfort and prevent the influence on the outer appearance, the thickness of the absorber 4 is less than or equal to 5 mm and preferably between 2 mm and 5 mm. Further, the density (average density) of the absorber is preferably greater than or equal to 0.01 g/cm³.

Synthetic fibers may also be added to the absorber 4. The synthetic fibers may be made of, for example, polyolefin such as polyethylene or polypropylene, polyester such as polyethylene terephthalate or polybutylene terephthalate, polyamide such as nylon, or a copolymer of these polymers. Also, a mixture of two types of these synthetic fibers may be used. Also, bicomponent fibers may be used for the synthetic fibers. Examples of bicomponent fibers include a core-in-sheath fiber including a high-melting-point fiber as a core and a low-melting-point fiber as a sheath, a side-by-side fiber, and a split fiber. When using hydrophobic fibers as the synthetic fibers, the hydrophobic fibers are preferably surface-treated with a hydrophilic agent to have an affinity to the body fluid.

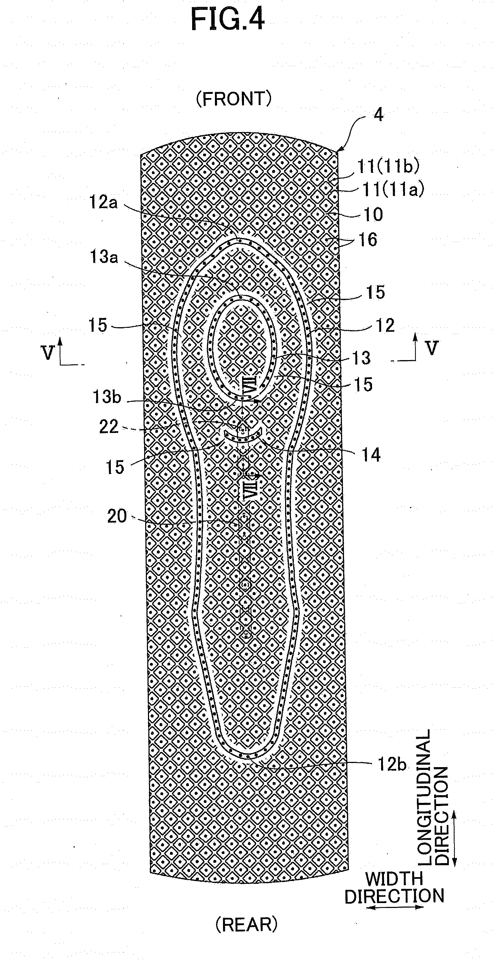

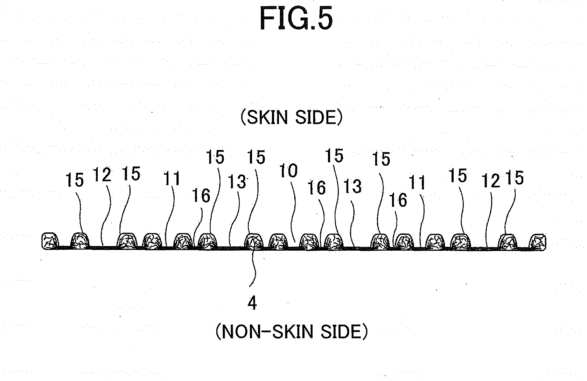

As illustrated in FIGs. 4 and 5, a core press 10 is formed on at least a surface (skin side surface) of the absorber 4 facing the upper-side sheet 3 by depressing the absorber 4 toward the back-side sheet 2 (non-skin side) such that spaces are formed between the absorber 4 and the upper-side sheet 3. The core press 10 is formed to reduce the thickness of the absorber 4 in the thickness direction. The core press 10 is formed by compressing the absorber 4 in a predetermined pattern at predetermined intervals instead of compressing the entire surface of the absorber 4. Because core-press-formed portions of the absorber 4 where the core press 10 is formed are compressed in the thickness direction, fibers in portions where the core press 10 is not formed are drawn by the core-press-formed portions, and the portions where the core press 10 is not formed are influenced and compressed to some extent. As a result, the thickness of the entire absorber 4 is reduced in the thickness direction. The "thickness of the absorber 4" is the thickness of the entire absorber 4 and specifically indicates the maximum thickness measured at the thickest portion. For example, the thickness may be measured with a constant pressure thickness gauge Digital Type FFD-7 manufactured by Ozaki Mfg. Co., Ltd.

When the thickness of the absorber 4 before forming the core press 10 is to and the thickness of the absorber 4 after the core press 10 is formed is t, the ratio t/to of the thickness reduced by forming the core press 10 is preferably between 1/10 and 7/10. If the ratio is less than 1/10, the absorber becomes hard and the wearing comfort is reduced. On the other hand, if the ratio is greater than 7/10, the effect of compression by the core press 10 becomes small and the thickness of the absorber 4 increases. The ratio may be changed by adjusting, for example, the compression depth of the core press 10, the interval between adjacent core presses, and the plane pattern of the core press 10.

In the illustrated example, the core press 10 is formed by compressing the absorber 4 only from the skin side (the surface facing the upper-side sheet 3). As a result, U-shaped recesses are formed on the skin side of the absorber 4, and the non-skin side (the surface facing the back-side sheet 2) of the absorber 4 becomes substantially flat. Such processing may be performed by passing the absorber through a gap between a patterned roller having multiple protrusions on its surface and an anvil roller having a flat surface. On the other hand, although not shown, U-shaped recesses may be formed on both of the skin side and the non-skin side of the absorber 4 by compressing the absorber 4 from both of the skin side and the non-skin side. Such processing may be performed by passing the absorber 4 through a gap between a first patterned roller having multiple protrusions on its surface and a second patterned roller having protrusions formed at positions corresponding to the protrusions of the first patterned roller. When at least the skin side of the absorber 4 is compressed, a body fluid entering through the upper-side sheet 3 flows into the recesses of the core press 10, can smoothly diffuse along the core press 10, and can be absorbed by the absorber 4 while diffusing.

As illustrated in FIG. 4, the core press 10 is preferably formed in the same pattern on substantially the entire absorber 4. That is, the core press 10 is also formed in the area H corresponding to the body fluid discharge part of the wearer in the same pattern as in the other areas. This makes it possible to reduce the thickness of the entire absorber 4, to reliably reduce the wearing discomfort, and to effectively reduce the influence on the outer appearance.

The core press 10 can be formed in any pattern as long as the thickness of the absorber 4 can be reduced in the thickness direction. However, as illustrated in FIG. 4, the core press 10 preferably includes multiple linear pressed parts 11 that are continuous or intermittent in a predetermined portion and formed between edges of the absorber 4. The linear pressed parts 11 are not formed in a middle portion that does not reach the outer edges of the absorber 4. Instead, each linear pressed part 11 is formed between one of the front, rear, right, and left edges of the absorber 4 and another one of the front, rear, right, and left edges. Forming the linear pressed parts 11 between the edges of the absorber 4 makes it possible to compress the entire absorber 4 and to effectively reduce its thickness.

The core press 10 preferably includes a grid-like pattern composed of first linear pressed parts 11a that are inclined to one side in the width direction and arranged in the longitudinal direction at predetermined intervals and second linear pressed parts 11b that are inclined to the other side in the width direction to intersect the first linear pressed parts 11a and arranged in the longitudinal direction at predetermined intervals. Forming the core press 10 in a grid-like pattern makes it possible to more reliably reduce the thickness of the absorber 4, and to more effectively reduce the wearing discomfort and suppress the influence on the outer appearance. The first linear pressed parts 11a and the second linear pressed parts 11b, respectively, are preferably arranged at substantially the same interval such that a large number of substantially rectangular cells having a square or rhombic shape and defined by the linear pressed parts 11a and 11b are arranged. Although the substantially rectangular cells surrounded by the linear pressed parts 11a and 11b are not directly compressed, their thickness is influenced by the linear pressed parts 11a and 11b and is made smaller than the original thickness.

The intersecting angle between the first linear pressed parts 11a and the second linear pressed parts 11b is substantially a right angle in the illustrated example. However, the intersecting angle may be between about 30 degrees and about 150 degrees. The first linear pressed parts 11a and the second linear pressed parts 11b are preferably inclined with respect to the width direction of the sanitary napkin 1. With this configuration, when the right and left legs are moved back and forth during, for example, walking and a force is applied to the sanitary napkin 1 in a torsional direction due to a pressure from the inside of the groin, the absorber 4 can smoothly deform in an oblique direction along the linear pressed parts 11a and 11b and can smoothly follow the movement of the body. Also, because the body fluid flows in the width direction of the sanitary napkin 1 along the linear pressed parts 11a and 11b, side leakage can be prevented compared with a case where linear pressed parts are formed to extend parallel to the width direction. To facilitate deformation in the oblique direction and prevent side leakage as described above, the linear pressed parts 11a and 11b are preferably inclined at an angle greater than or equal to 30 degrees with respect to the width direction of the sanitary napkin 1.

To reliably reduce the thickness of the absorber 4, as illustrated in FIG. 4, a length "a" of each side of the cells defined by the first linear pressed parts 11a and the second linear pressed parts 11b is preferably between 4 mm and 10 mm. If the length "a" is less than 4 mm, the distance between the linear pressed parts 11a and 11b becomes too close. As a result, the absorber 4 becomes hard, and the wearing comfort is reduced. On the other hand, if the length "a" is greater than 10 mm, the central portion of each cell bulges toward the skin side, and the thickness of the entire absorber 4 cannot be sufficiently reduced.

To reliably reduce the thickness of the absorber 4, as illustrated in FIGs. 4 and 5, a central pressed part 16 shaped like, for example, a circular or polygonal dot may be formed in the central portion of each cell which is apart from the linear pressed parts 11a and 11b. This configuration makes it possible to suppress the central portion of each cell from bulging toward the skin side, and makes it possible to effectively reduce the thickness of the entire absorber 4 without much increasing the hardness of the absorber 4. In addition, the body fluid penetrated into the cell can smoothly diffuse toward the central pressed part 16 with a high fiber density due to the capillary action. This in turn improves the diffusivity and makes it possible to quickly absorb the body fluid. When the central pressed part 16 is formed, the length "a" of each side of the cell may be made greater than the length in a case where the central pressed part 16 is not formed. For example, even if the length "a" is increased up to about 15 mm, the thickness of the absorber 4 can be reduced without problem. The width of each of the linear pressed parts 11a and 11b is preferably between 0.5 mm and 3 mm.

Alternatively, the linear pressed parts 11a and 11b may be arranged in the width direction and the longitudinal direction of the absorber 4 to form a regular grid pattern. Also, the linear pressed parts 11a and 11b may be arranged to form a honeycomb pattern composed of hexagonal cells. Any pattern may be used as long as the thickness of the absorber 4 becomes less than or equal to 5 mm and the body fluid can smoothly diffuse inside of the absorber 4. In the above embodiment, the linear pressed parts 11a and 11b are formed as continuous lines. However, the linear pressed parts 11a and 11b may be formed as discontinuous lines composed of multiple dots arranged at intervals.

As illustrated in FIGs. 4 and 5, on the surface of the absorber 4 facing the upper-side sheet 3, in addition to the core press 10, an outer absorber embossment 12, an inner absorber embossment 13, and a lateral absorber embossment 14 are formed. The outer absorber embossment 12 is long in the longitudinal direction in plan view and is formed by depressing the absorber 4 toward the back-side sheet 2 such that a space is formed between the absorber 4 and the upper-side sheet 3. The front part of the outer absorber embossment 12 surrounds the area H corresponding to the body fluid discharge part of the wearer, and the rear part of the outer absorber embossment 12 has an elongated shape and extends backward to surround an area corresponding to the buttock groove of the wearer. The inner absorber embossment 13 is disposed inside of the outer absorber embossment 12 and has a long elliptical shape that particularly surrounds an area corresponding to the vaginal opening of the wearer in the area H corresponding to the body fluid discharge part of the wearer. The lateral absorber embossment 14 is disposed behind and apart from the inner absorber embossment 13 and is formed in the center of the absorber 4 in the width direction to extend substantially in the width direction.

The outer absorber embossment 12 prevents the body fluid from rapidly diffusing outward, thereby prevents leakage from the ends of the absorber 4, and makes the area surrounded by the embossment readily fit the skin surface.

The inner absorber embossment 13 receives the body fluid discharged from the vaginal opening and reduces the speed of diffusion to the outside.

In the planar shapes of the outer absorber embossment 12 and the inner absorber embossment 13 exemplified in the figures, the front part of the outer absorber embossment 12 has an elliptical shape that is long in the napkin longitudinal direction, the rear part of the outer absorber embossment 12 has an elongated shape, and the inner absorber embossment 13 has an elliptical shape that is long in the napkin longitudinal direction. However, the present invention is not limited to this example. For example, to reduce only the outward diffusion of the body fluid in the width direction, the embossments may be formed as curved lines extending in the longitudinal direction on the sides of the body fluid discharge part corresponding area, or the outer absorber embossment 12 may be formed in a vertically elongated elliptical shape similar to the inner absorber embossment 13 without extending the outer absorber embossment 12 to the area corresponding to the buttock groove. A part or the entirety of one or both of the outer absorber embossment 12 and the inner absorber embossment 13 may be implemented by multiple lines that are arranged at predetermined intervals. Also, one or both of the outer absorber embossment 12 and the inner absorber embossment 13 may be omitted.

As described later in more detail, the lateral absorber embossment 14 causes the body fluid flowing from a longitudinal surface embossment 20 described later to diffuse in the width direction and facilitates the absorption of the body fluid into the absorber 4.

The lateral absorber embossment 14 is formed by compressing only the absorber 4 or both of the absorber 4 and the enveloping sheet 5 from the skin side of the absorber 4 or the enveloping sheet 5 such that a space if formed between the upper-side sheet 3 and the absorber 4 or the enveloping sheet 5. This makes it possible to prevent the upper-side sheet 3 from being altered into a film when the upper-side sheet 3 is compressed together with the absorber 4 while heating the upper-side sheet 3, and thereby makes it possible to prevent the blockage of the transfer of the body fluid from the upper-side sheet 3 to the absorber 4.

Similarly to the core press 10, the lateral absorber embossment 14 may be implemented by a U-shaped recess formed only on the skin side of the absorber 4 by compressing the absorber 4 only from the skin side, or may be implemented by U-shaped recesses formed on both of the skin side and the non-skin side of the absorber 4 by compressing the absorber 4 from both of the skin side and the non-skin side.

Also, the lateral absorber embossment 14 may be formed in various manners. Preferably, as illustrated in FIG. 4, the lateral absorber embossment 14 is disposed behind and apart from the inner absorber embossment 13 and apart from the outer absorber embossment 12, and has a curved planar shape that protrudes backward. When the longitudinal surface embossment 20 is formed to cross front and rear portions 13a and 13b of the inner absorber embossment 13 extending substantially in the width direction of the absorber 4, the front and rear portions 13a and 13b of the inner absorber embossment 13 may be regarded as lateral absorber embossments 14. This is because, when the longitudinal surface embossment 20 crosses the front and rear portions 13a and 13b, the portions 13a and 13b also provide a function that is the same as the function of the lateral absorber embossment 14. However, it is not preferable to use portions of the outermost embossment, in the example illustrated in FIG. 4, front and rear portions 12a and 12b of the outer absorber embossment 12 extending substantially in the width direction of the absorber 4, as the lateral absorber embossment 14. Because the outer absorber embossment 12 is provided to reduce the diffusion of the body fluid to the outside of the outer absorber embossment 12 and to prevent leakage from the ends of the absorber 4, if the longitudinal surface embossment 20 is provided to cross the outer absorber embossment 12, the body fluid tends to diffuse outside of the outer absorber embossment 12, and the effect of the outer absorber embossment 12 may be reduced.

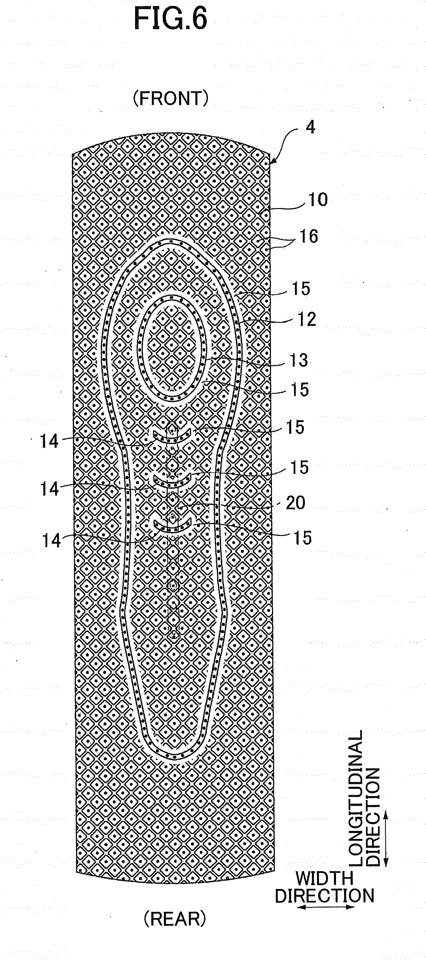

As illustrated in FIG. 6, multiple lateral absorber embossments 14 may be arranged at intervals in the longitudinal direction of the sanitary napkin 1. With multiple lateral absorber embossments 14, the flow velocity and the flow rate of the body fluid flowing through the longitudinal surface embossment 20 gradually decrease each time the body fluid passes through the lateral absorber embossment 14. Thus, this configuration makes it possible to prevent the body fluid from rapidly flowing into the rear end of the longitudinal surface embossment 20. The number of the lateral absorber embossments 14 is between 1 and 10 and preferably between 2 and 5. When the distance between adjacent lateral absorber embossments 14 is too short, the fluid diffusion is hampered; and when the distance is too long, the effect of the fluid diffusion is reduced. For this reason, the distance is preferably between about 10 mm and about 30 mm.

The planar shape of the lateral absorber embossment(s) 14 is preferably, but is not limited to, a curved line that protrudes toward the rear end of the absorber 4 as illustrated in FIG. 4. With this configuration, the body fluid flowing through the longitudinal surface embossment 20 from the front side to the rear side is more likely to flow into the lateral absorber embossment 14, and the body fluid flown into the lateral absorber embossment 14 is more likely to contact the side surfaces of the lateral absorber embossment 14 (i.e., the side surfaces of a groove of the lateral absorber embossment 14) instead of flowing straight to the lateral ends and is more likely to be absorbed by the absorber 4 through the side surfaces. The planar shape of the lateral absorber embossment 14 may also be a straight line extending substantially in the width direction of the absorber 4 or a curved line protruding forward.

The length of the lateral absorber embossment 14 in the width direction of the absorber 4 is not limited to any specific value. Increasing this length increases the diffusivity in the width direction of the absorber 4 and may cause leakage from the lateral edges of the absorber 4. Therefore, it is not preferable to excessively increase this length. Although it varies depending on the width of the absorber 4 and the groove width of the longitudinal surface embossment 20, this length may be between 1/10 and 1/2 and preferably between 1/7 and 1/3 of the width of the absorber 4.

As illustrated in FIGs. 4 and 5, the core press 10 is preferably omitted in positions where the core press 10 overlaps the lateral absorber embossment 14 and in the vicinity of the lateral absorber embossment 14 such that a non-pressed part 15 with a predetermined width is formed around the lateral absorber embossment 14. That is, in the lateral absorber embossment 14 and an area having a predetermined width and surrounding the lateral absorber embossment 14, the core press 10 is not formed, and an uncompressed area (the non-pressed part 15) is formed between the lateral absorber embossment 14 and the core press 10. With the non-pressed part 15 formed around the lateral absorber embossment 14, the body fluid is quickly absorbed from the lateral absorber embossment 14 into the non-pressed part 15 having a large inter-fiber gap, and the body fluid penetrates into the high density core press 10 after being temporarily retained in the non-pressed part 15. Thus, this configuration enables the body fluid to be quickly absorbed into the absorber 4. Also, because the body fluid once absorbed by the absorber 4 is less likely to move to the non-pressed part 15 with a low density, this configuration makes it possible to prevent the body fluid from returning to the lateral absorber embossment 14.

The non-pressed part 15 is formed around the entire periphery of the lateral absorber embossment 14 so that the lateral absorber embossment 14 and the core press 10 are not directly connected to each other. The width of the non-pressed part 15 is preferably between 0.5 mm and 5 mm to ensure its function as a body fluid buffer zone and to prevent the thickness of the absorber 4 from increasing.

The non-pressed part 15 is preferably formed also around the outer absorber embossment 12 and the inner absorber embossment 13.

In forming the core press 10, the outer absorber embossment 12, the inner absorber embossment 13, and the lateral absorber embossment 14 on the absorber 4, the core press 10 and the embossments 12, 13, and 14 may be formed simultaneously using a patterned roll on which protrusions corresponding to the core press 10 and the embossments 12, 13, and 14 are formed. Alternatively, the embossments 12, 13, and 14 may be formed after the core press 10 is formed. The core press 10, the central pressed part 16, and the embossments 12, 13, and 14 are preferably formed with substantially the same groove depth to make the bottom compression density substantially the same, to make the body fluid drawing force due to the capillary action substantially the same, and to prevent unbalanced body fluid distribution.

### <LONGITUDINAL SURFACE EMBOSSMENT>

In the sanitary napkin 1, as illustrated in FIG. 1, the longitudinal surface embossment 20 is formed on the outer surface of the upper-side sheet 3 by depressing the upper-side sheet 3 and the absorber 4 together toward the back-side sheet 2. The longitudinal surface embossment 20 is disposed in the central portion in the width direction of the sanitary napkin 1 and extends in the longitudinal direction to cross the lateral absorber embossment 14. The longitudinal surface embossment 20 causes the body fluid in the area H corresponding to the body fluid discharge part to diffuse in the width direction while diffusing toward the rear side of the absorber 4 so that the wide area of the absorber 4 can be efficiently used.

The longitudinal surface embossment 20 is preferably formed to extend from the area H corresponding to the body fluid discharge part of the wearer toward the rear side of the sanitary napkin 1. This configuration makes it possible to cause the body fluid in the area corresponding to the body fluid discharge part to diffuse backward, and thereby makes it possible to efficiently use a wide area of the absorber. The longitudinal surface embossment 20 is preferably formed from the area H corresponding to the body fluid discharge part of the wearer, and more specifically, from a position immediately behind the vaginal opening. That is, the longitudinal surface embossment 20 is preferably formed to overlap the area H corresponding to the body fluid discharge part of the wearer. In the embodiment illustrated in FIG. 1, the longitudinal surface embossment 20 is disposed behind the inner absorber embossment 13 at a distance.

The rear end of the longitudinal surface embossment 20 preferably extends up to the middle of the area corresponding to the buttock groove of the wearer. If the longitudinal surface embossment 20 is extended to the rear side of the absorber 4, leakage from the rear end of the absorber 4 may occur. Specifically, the longitudinal surface embossment 20 is preferably extended up to a position that slightly exceeds, in the backward direction, the longitudinal center of the rear part of the outer absorber embossment 12 formed to surround the buttock groove.

If the groove width of the longitudinal surface embossment 20 is too narrow, the body fluid cannot smoothly flow; and if the groove width of the longitudinal surface embossment 20 is too wide, the wearer may feel discomfort. Therefore, the groove width of the longitudinal surface embossment 20 is preferably between 2 mm and 10 mm. The groove width of the longitudinal surface embossment 20 may be substantially constant throughout the entire length, or may be changed in the middle. As an example where the width is changed, the groove width may be made relatively wide in a portion crossing the lateral absorber embossment 14 to facilitate the flow of the body fluid into the lateral absorber embossment 14. Also, because the body fluid flowing through the longitudinal surface embossment 20 flows into the lateral absorber embossment 14 and the flow rate of the body fluid in the longitudinal surface embossment 20 decreases each time the longitudinal surface embossment 20 crosses the lateral absorber embossment 14, the groove width of the longitudinal surface embossment 20 may be gradually decreased toward the rear side of the absorber 4 or each time the longitudinal surface embossment 20 crosses the lateral absorber embossment 14. This configuration makes it possible to reduce the groove width in the rear side and thereby makes it possible to reduce the wearing discomfort.

As illustrated in FIG. 1, the longitudinal surface embossment 20 preferably includes low-compressed parts 21 and high-compressed parts 22, and the high-compressed parts 22 are preferably arranged at intervals in the direction in which the longitudinal surface embossment 20 extends. The low-compressed parts 21 are portions having a relatively shallow groove depth, and the high-compressed parts 22 are portions having a relatively deep groove depth. In FIG. 1, the high-compressed parts 22 are represented by black parts, and the low-compressed parts 21 are represented by white parts. With the low-compressed parts 21 and the high-compressed parts 22, because the groove depth increases at the high-compressed parts 22, the flow of the body fluid flowing along the bottom of the embossed groove changes abruptly and as a result, the flow rate is reduced.

Also, some of the high-compressed parts 22 preferably overlap at least a part of the non-pressed part 15. That is, the longitudinal surface embossment 20 is preferably formed such that the high-compressed parts 22 overlap the non-pressed part 15. With this configuration, the non-pressed part 15, which is not compressed and has a large restoring force, is prevented from returning to its original state, the high-compressed part 22 can reliably maintain the compressed state, and the longitudinal surface embossment 20 can be formed reliably. Also, with the high-compressed parts 22 disposed to overlap the non-pressed part 15, the body fluid absorbed into the non-pressed part 15 is likely to be drawn into the high-compressed parts 22 due to the capillary action. This makes it possible to improve the absorption rate of the body fluid.

To prevent the high-compressed part 22 from becoming hard and prevent the wearing comfort from being reduced, the area of the high-compressed part 22 is preferably less than or equal to 1 cm². Although the planar shape of the high-compressed part 22 is not limited to any specific shape, the length in the longitudinal direction of the embossed groove is preferably 1.5 times or more greater than the length in the width direction of the embossed groove to facilitate the flow of the body fluid in the longitudinal direction of the embossed groove. In the example illustrated in FIG. 1, the high-compressed part 22 has a substantially rhombic shape that is long in the longitudinal direction of the embossed groove.

The distance between adjacent high-compressed parts 22 is preferably between 2 mm and 20 mm. If the distance is less than 2 mm, the body fluid is less likely to penetrate into the non-pressed part 15; and if the distance is greater than 20 mm, the flow rate reduction effect of the high-compressed parts 22 is reduced.

The high-compressed part 22 is preferably provided at least near the front side of the lateral absorber embossment 14. With this configuration, the body fluid flowing through the longitudinal surface embossment 20 from the front side to the rear side reaches the lateral absorber embossment 14 immediately after its flow rate is reduced by the high-compressed part 22. This facilitates the flow of the body fluid into the lateral absorber embossment 14. Also, the high-compressed part 22 serves as a base point that causes the body fluid flowing through the longitudinal surface embossment 20 to disperse into the lateral absorber embossment 14. Specifically, the high-compressed part 22 is preferably disposed such that the rear end of the high-compressed part 22 overlaps the non-pressed part 15 located adjacent to the front side of the lateral absorber embossment 14.

Preferably, the groove depth of the low-compressed part 21 is substantially the same as or slightly greater than the groove depth of the core press 10 and the embossments 12, 13, and 14 formed on the absorber 4.

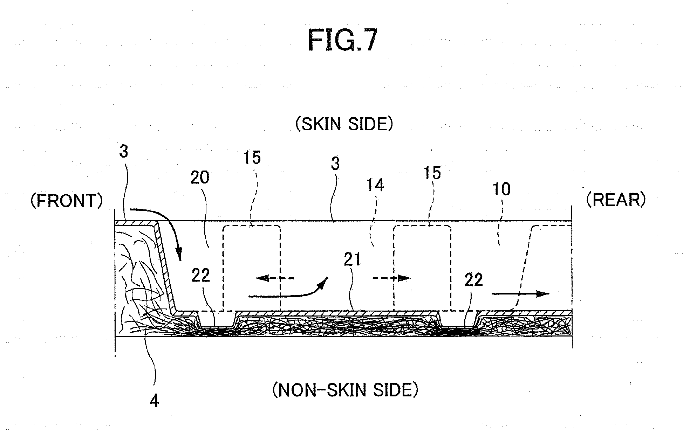

The flow of the body fluid in the sanitary napkin 1 configured as described above is described in detail with reference to FIG. 7. The body fluid discharged into the area H corresponding to the body fluid discharge part of the wearer flows into the longitudinal surface embossment 20, and flows in the groove of the longitudinal surface embossment 20 toward the rear side. While flowing toward the rear side, the body fluid penetrates the upper-side sheet 3 from the side surfaces of the longitudinal surface embossment 20 and flows into the lateral absorber embossment 14. Because the non-pressed part 15 with a low density and a large inter-fiber gap is formed around the lateral absorber embossment 14, the body fluid flown into the lateral absorber embossment 14 contacts the fibers constituting the non-pressed part 15 and is absorbed into the gaps between the fibers of the non-pressed part 15 due to the capillary action. Then, the body fluid absorbed into the non-pressed part 15 is drawn into the adjacent core press 10 with a small inter-fiber gap due to the capillary action, and permeates into and is retained in the cells defined by the core press 10. Also, the flow rate of the body fluid flowing toward the rear side through the longitudinal surface embossment 20 is gradually decreased by the high-compressed parts 22 formed in the bottom; and the body fluid penetrates the upper-side sheet 3 from the side surfaces, is absorbed into the absorber 4, and is retained in the cells defined by the core press 10 and the core press 10 itself. Thus, the body fluid flows from the longitudinal surface embossment 20 into the lateral absorber embossment 14, and is quickly absorbed and held in the absorber 4. This configuration makes it possible to cause the body fluid to be diffused and absorbed in a wide area of the absorber 4 without lowering the absorption rate, and thereby makes it possible to efficiently use the absorption capacity of the absorber. Also, this configuration makes it possible to decrease the flow rate of the body fluid flowing through the longitudinal surface embossment 20, prevent the body fluid from flowing rapidly toward the rear side of the longitudinal surface embossment 20, and prevent the rear-side leakage of the body fluid reaching the rear end.

Preferred embodiments of the present invention are described below as appendices.

### (Appendix 1)

According to an embodiment of Appendix 1, an absorbent article includes an absorber disposed between a permeable upper-side sheet and an impermeable back-side sheet. A core press and a lateral absorber embossment are formed at least on a surface of the absorber facing the upper-side sheet, the core press is formed by depressing the absorber toward the back-side sheet such that spaces are formed between the absorber and the upper-side sheet and the thickness of the absorber is reduced in the thickness direction, the lateral absorber embossment is formed in a central portion in the width direction of the absorber and extends substantially in the width direction, a non-pressed part is formed along the periphery of the lateral absorber embossment by omitting the core press in a position where the core press overlaps the lateral absorber embossment and in the vicinity of the lateral absorber embossment, and a longitudinal surface embossment is formed on an outer surface of the upper-side sheet by depressing the upper-side sheet and the absorber together toward the back-side sheet such that the longitudinal surface embossment extends in the longitudinal direction in a central portion in the width direction of the absorbent article and crosses the lateral absorber embossment.

The embodiment of Appendix 1 provides a slim absorbent article in which the thickness of an absorber is reduced in the thickness direction by forming a core press on the absorber. Forming the core press makes it possible to form fiber density differences in the absorber and to improve the diffusivity inside of the absorber. Also, forming the longitudinal surface embossment along the longitudinal direction in the central portion in the width direction of the absorbent article enables the body fluid to flow inside of the longitudinal surface embossment and makes it possible to improve the diffusivity in the longitudinal direction of the absorbent article. Here, because the bottom of the longitudinal surface embossment is compacted by the compression and has a very high fiber density, the body fluid absorption performance tends to be low. Also, in portions of the side surfaces of the longitudinal surface embossment where the core press is formed, fibers are compacted by the core press, and the body fluid is not readily absorbed. However, in the present invention, because the longitudinal surface embossment is formed to cross the lateral absorber embossment, a part of the body fluid flowing through the longitudinal surface embossment flows into the lateral absorber embossment and is absorbed into the absorber at the lateral absorber embossment. Because the non-pressed part with a small fiber density and a large inter-fiber gap is formed around the lateral absorber embossment, the body fluid can smoothly penetrate into the lateral absorber embossment through the side surface. The body fluid penetrated into the non-pressed part diffuses in the width direction along the lateral absorber embossment and can smoothly diffuse to a wide area of the absorber due to the capillary action caused by the fiber density difference with the core press. Also, the body fluid penetrated into the absorber with a small inter-fiber gap from the non-pressed part is less likely to move to the non-pressed part with a large inter-fiber gaps and is therefore less likely to return to the lateral absorber embossment. Thus, in a slim absorbent article using a thin absorber, the present embodiment makes it possible to absorb and retain a greater amount of body fluid by efficiently using a wide area of the absorber without causing a decrease in the absorption rate and leakage

### (Appendix 2)

An embodiment of Appendix 2 provides the absorbent article described in Appendix 1 where the longitudinal surface embossment includes low-compressed parts and high-compressed parts, and the high-compressed parts are arranged at intervals in a longitudinal direction of the longitudinal surface embossment.

According to the embodiment of Appendix 2, the longitudinal surface embossment includes the low-compressed parts and the high-compressed parts. With this configuration, when the body fluid passes through the high-compressed parts whose bottom surfaces are recessed, the flow rate of the body fluid decreases temporarily, and the rapid flow of the body fluid through the longitudinal surface embossment is prevented.

### (Appendix 3)

An embodiment of Appendix 3 provides the absorbent article described in Appendix 1 or 2 where the longitudinal surface embossment is formed to extend rearward from an area corresponding to a body fluid discharge part of a wearer.

According to the embodiment of Appendix 3, the longitudinal surface embossment is formed to extend rearward from the area corresponding to the body fluid discharge part of the wearer. This configuration enables the body fluid discharged in this area to smoothly diffuse to the rear side of the absorbent article.

### (Appendix 4)

An embodiment of Appendix 4 provides the absorbent article described in any one of Appendices 1-3 where multiple lateral absorber embossments are arranged at intervals in the longitudinal direction of the absorbent article.

With the embodiment of Appendix 4 where multiple lateral absorber embossments are arranged at intervals in the longitudinal direction of the absorbent article, the body fluid flows into the lateral absorber embossment while flowing through the longitudinal surface embossment, and the flow rate of the body fluid gradually decreases toward the rear side. This configuration makes it possible to prevent the body fluid from flowing rapidly to the rear end of the longitudinal surface embossment, and makes it possible to prevent the leakage from the rear end of the absorber.

### (Appendix 5)

An embodiment of Appendix 5 provides the absorbent article described in any one of Appendices 1-4 where the lateral absorber embossment is implemented by one or both of a substantially-laterally extending portion of an annular compressed groove formed around the area corresponding to the body fluid discharge part of the wearer and an independent compressed groove extending substantially in the width direction of the absorbent article.

The embodiment of Appendix 5 defines that the lateral absorber embossment may be implemented by a substantially-laterally extending portion of an annular compressed groove formed around the area corresponding to the body fluid discharge part of the wearer or an independent compressed groove. In either case, the body fluid flowing through the longitudinal surface embossment can flow into the lateral absorber embossment and can be diffused, absorbed, and retained in a wide area.

### (Appendix 6)

An embodiment of Appendix 6 provides the absorbent article described in any one of Appendices 1-5 where the thickness of the absorber is less than or equal to 5 mm.

In the embodiment of Appendix 6, the thickness (maximum thickness) of the absorber is reduced to 5 mm or less by forming the core press on the absorber.

The present application is based on and claims priority to Japanese Patent Application No. 2018-055517 filed on March 23, 2018, the entire contents of which are hereby incorporated herein by reference.

### EXPLANATION OF REFERENCE NUMERALS

1 ... sanitary napkin, 2 ... back-side sheet, 3 ... upper-side sheet, 4 ... absorber, 5 ... enveloping sheet, 7 ... side nonwoven fabric, 10 ... core press, 11 ... linear pressed part, 11a ... first linear pressed part, 11b ... second linear pressed part, 12 ... outer absorber embossment, 13 ... inner absorber embossment, 14 ... lateral absorber embossment, 15... non-pressed part, 20 ... longitudinal surface embossment, 21 ... low-compressed part, 22 ... high-compressed part

## Claims

1. An absorbent article, comprising:
an absorber disposed between a permeable upper-side sheet and an impermeable back-side sheet, wherein
a core press and a lateral absorber embossment are formed at least on a surface of the absorber facing the upper-side sheet;
the core press is formed by depressing the absorber toward the back-side sheet such that spaces are formed between the absorber and the upper-side sheet and a thickness of the absorber is reduced in a thickness direction;
the lateral absorber embossment is formed in a central portion in a width direction of the absorber and extends substantially in the width direction;
a non-pressed part is formed along a periphery of the lateral absorber embossment by omitting the core press in a position where the core press overlaps the lateral absorber embossment and in a vicinity of the lateral absorber embossment; and
a longitudinal surface embossment is formed on an outer surface of the upper-side sheet by depressing the upper-side sheet and the absorber together toward the back-side sheet such that the longitudinal surface embossment extends in a longitudinal direction in a central portion in a width direction of the absorbent article and crosses the lateral absorber embossment.

2. The absorbent article as claimed in claim 1, wherein
the longitudinal surface embossment includes low-compressed parts and high-compressed parts; and
the high-compressed parts are arranged at intervals in a longitudinal direction of the longitudinal surface embossment.

3. The absorbent article as claimed in claim 1, wherein the longitudinal surface embossment is formed to extend rearward from an area corresponding to a body fluid discharge part of a wearer.

4. The absorbent article as claimed in claim 1, wherein multiple lateral absorber embossments are arranged at intervals in a longitudinal direction of the absorbent article.

5. The absorbent article as claimed in claim 1, wherein the lateral absorber embossment is implemented by one or both of a substantially-laterally extending portion of an annular compressed groove formed around an area corresponding to a body fluid discharge part of a wearer and an independent compressed groove extending substantially in the width direction of the absorbent article.

6. The absorbent article as claimed in claim 1, wherein a thickness of the absorber is less than or equal to 5 mm.
